(19) Europäisches Patentamt
European Patent Office
Office européen des brevets

(11) Veröffentlichungsnummer : **0 164 706**
**B1**

(12) # EUROPÄISCHE PATENTSCHRIFT

(45) Veröffentlichungstag der Patentschrift : **05.04.89**

(21) Anmeldenummer : **85107064.9**

(22) Anmeldetag : **07.06.85**

(51) Int. Cl.[4] : **C 07 D265/30**, C 07 D295/02, C 07 D295/06, C 07 D295/08, C 07 D211/14, C 07 D279/12, A 01 N 43/84, A 01 N 43/40// C07C21/24, C07C25/06, C07C25/02, C07C25/10, C07C25/08

(54) **Phenylalkylamine - Bioregulatoren.**

(30) Priorität : **12.06.84 DE 3421810**

(43) Veröffentlichungstag der Anmeldung : **18.12.85 Patentblatt 85/51**

(45) Bekanntmachung des Hinweises auf die Patenterteilung : **05.04.89 Patentblatt 89/14**

(84) Benannte Vertragsstaaten : **AT BE CH DE FR GB IT LI NL SE**

(56) Entgegenhaltungen :
**EP-A- 0 002 604**
**EP-A- 0 007 479**
**EP-A- 0 024 334**
**CHEMICAL ABSTRACTS, Band 94, Nr. 21, 25. Mai 1981, Seite 735, Nr. 175136s, Columbus, Ohio, US; DD - A - 144 148 (FRANKE, FRIEDRICH et al.) 01.10.1980**

(73) Patentinhaber : **BASF Aktiengesellschaft**
**Carl-Bosch-Strasse 38**
**D-6700 Ludwigshafen (DE)**

(72) Erfinder : **Buschmann, Ernst, Dr.**
**Georg-Ludwig-Krebs-Strasse 10**
**D-6700 Ludwigshafen (DE)**
Erfinder : **Himmele, Walter, Dr.**
**Eichenweg 14**
**D-6909 Walldorf (DE)**
Erfinder : **Eckhardt, Heinz, Dr.**
**Ruedigerstrasse 7**
**D-6700 Ludwigshafen (DE)**
Erfinder : **Ernst, Hansgeorg, Dr.**
**Bruesseler Ring 38**
**D-6700 Ludwigshafen (DE)**
Erfinder : **Rademacher, Wilhelm, Dr.**
**Austrasse 1**
**D-6703 Limburgerhof (DE)**
Erfinder : **Jung, Johann, Prof. Dr.**
**Hardenburgstrasse 19**
**D-6703 Limburgerhof (DE)**

**EP 0 164 706 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

Es ist aus den DE-OSen 27 52 096, 26 56 747, 27 20 612, 27 53 278, 28 30 127 und der europäischen PS 5541 bekannt, daß Phenylpropylamine fungizid wirken. Das Pflanzenwachstum wird durch diese Verbindungen nur unwesentlich beeinflußt.

Es wurde nun gefunden, daß die Verlängerung des die beiden Ringstrukturen verbindenden Propylenrestes, die also zu Substanzen der Formel I führt

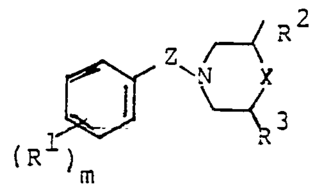

(I)

in der

$R^1$ bis zu drei gleiche oder verschiedene Substituenten, ausgewählt aus $CH_3$, $C_2H_5$, n-Propyl, iso-Propyl, n-Butyl, iso-Butyl, tert.-Butyl, tert.-Amyl, 1,1-Dimethyl-butyl, 1,1-Dimethyl-pentyl, Cyclopentyl, Cyclohexyl, Cycloheptyl, Norbornyl, Methoxy, Ethoxy, Propoxy, tert.-Butoxy, Acetyl, Propionyl, Benzoyl, Pivaloyl oder Halogen,

$R^2$ und $R^3$ Wasserstoff oder $C_1$- bis $C_3$-Alkyl,

z geradkettiges $C_5$- bis $C_{10}$-Alkylen, gegebenenfalls mit bis zu drei $C_1$- bis $C_4$-Alkyl substituiert, oder $CH_2—CH(CH_3)CH_2CH_2$,

X Sauerstoff, Schwefel, $(CH_2)_p$,

m die Zahlen 0 bis 3 und

p die Zahlen 0 bis 2 bedeuten,

Verbindungen liefert, die nicht mehr oder praktisch nicht mehr fungizid wirken, dafür aber überraschenderweise interessante wachstumsregulierende Wirkungen aufweisen. Zufällig aus der EP-A-0 002 604 bekannte Verbindungen dieser Klasse (Phenylbutylpiperidin, Phenylbutylpyrrolidin, Phenylbutylhexahydroazepin) sind bisher nur als pharmazartisch wirksam bekannt gewesen.

$R^1$ steht für die oben aufgeführten Reste. Halogen bedeutet F, Cl, Br oder J.

$R^2$ und $R^3$ bedeuten unabhängig voneinander Wasserstoff, Methyl, Ethyl, Propyl und Isopropyl.

z bedeutet beispielsweise $(CH_2)_4$, $CH_2CHCH_3CH_2CH_2$, $(CH_2)_5$, $CH_2CH_2CHCH_3CH_2CH_2$, $(CH_2)_3CHCH_3CH_2$, $CH_2CHCH_3CH_2CHCH_3CH_2$, $(CH_2)_6$, $(CH_2)_4CHCH_3CH_2$, $(CH_2)_7$, $(CH_2)_5CHCH_3CH_2$, $(CH_2)_8$, $(CH_2)_9$, $(CH_2)_{10}$.

Aminoreste in der Formel I sind beispielsweise die von Pyrrolidin, Piperidin, 3-Methylpiperidin, 3,5-Dimethylpiperidin, Morpholin, 2,6-cis-Dimethylmorpholin, 2,6-trans-Dimethylmorpholin, 2,6-Dimethylthiomorpholin, Hexamethylenimin abgeleiteten.

Bevorzugt sind Phenylalkylamine der Formel I, in der $R^2$ und $R^3$ jeweils Methyl und X Sauerstoff sind und $R^1$, z und m die in Anspruch 1 genannten Bedeutungen haben.

Die erfindungsgemäßen Verbindungen können als Wirkstoffe in Wachstumsregulatoren verwendet werden. Demgemäß beinhaltet die Erfindung ein Verfahren zur Regulierung des Pflanzenwachstums, das dadurch gekennzeichnet ist, daß man eine Verbindung der Formel I, in der $R^1$, $R^2$, X, m und p die oben genannten Bedeutungen haben und z geradkettiges $C_4$- bis $C_{10}$-Alkylen, gegebenenfalls mit bis zu drei $C_1$- bis $C_4$- Alkyl substituiert, auf die Pflanzen oder deren Vermehrungsgut einwirken läßt.

Wichtige Zwischenprodukte für die Herstellung der Phenylalkylamine i. S. der Erfindung sind die Phenylalkylhalogenide der Formel II

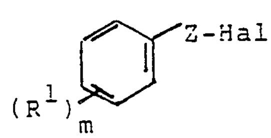

(II)

die ihrerseits beispielsweise durch Kondensationsreaktionen oder andere C—C-Verknüpfungsreaktion, wie in Schema 1 angegeben, hergestellt werden können.

(Siehe Schema 1 Seite 2 f.)

Schema 1

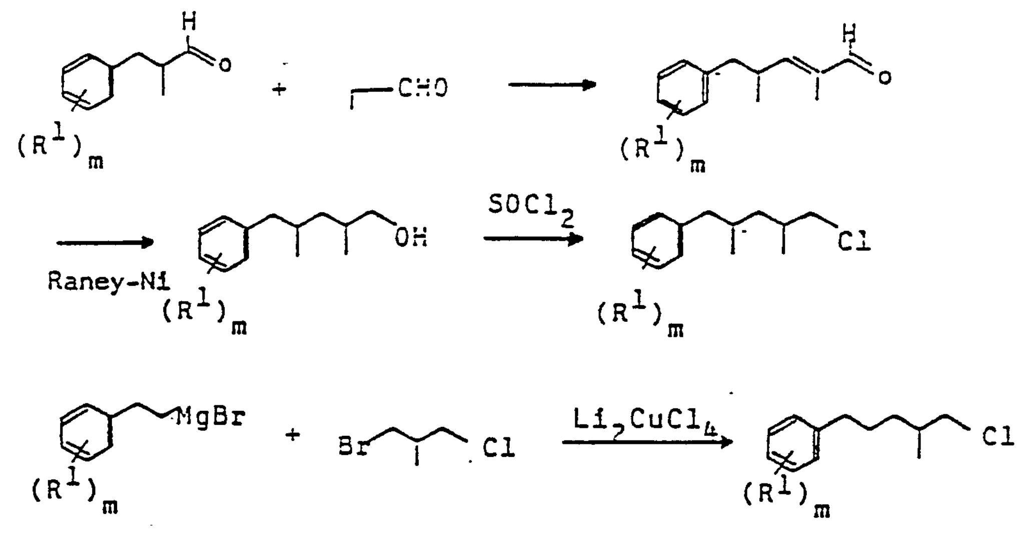


C—C-Verknüpfungen dieser Art sind z. B. beschrieben in Angew. Chem. 86, 50 (1974).

Eine bequeme Variation der Aromatensubstitution der Phenylalkylhalogenide ist möglich durch Umsetzung entsprechender bekannter, z. T. käuflicher Halogenide der Formel II (wobei $(R^1)_m$ auch Wasserstoff bedeuten kann).

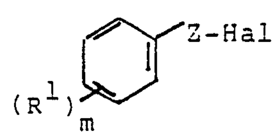
(II)

in der Halogen vorzugsweise Chlor, aber auch Brom oder Jod bedeutet.

Mit diesen Halogeniden ist das folgende Herstellverfahren möglich.

Schema 2

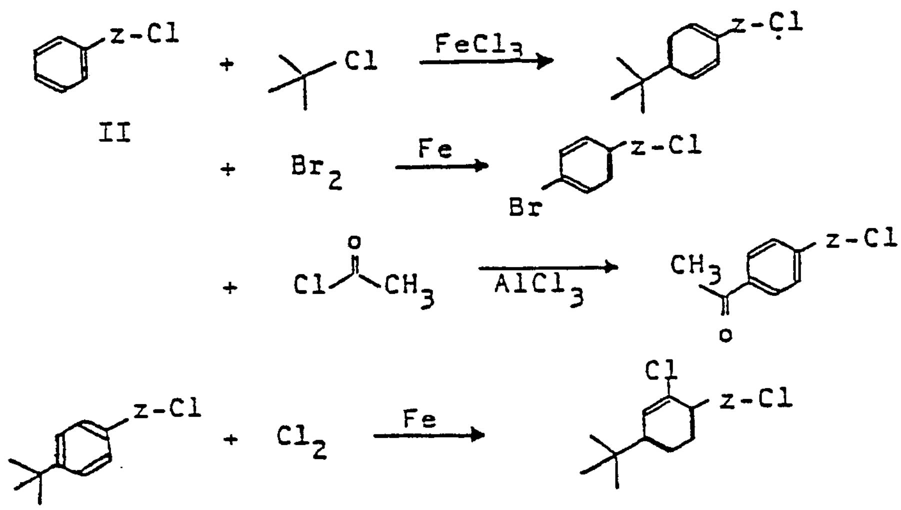


Die Synthese entsprechender Phenylpropylhalogenide, die in der europäischen PS 9077 beschrieben sind, können ohne Schwierigkeiten auch auf längerkettige Phenylalkylchloride übertragen werden.

· Umsetzung der entsprechenden Phenylalkylhalogenide (II)

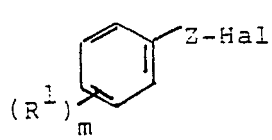
(II)

oder eines entsprechenden ω-Phenylalkanals der Formel

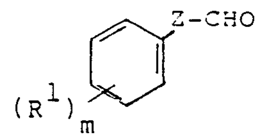

(III)

mit den sekundären cyclischen Aminen (IV)

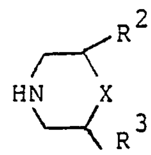

(IV)

führt zu den erfindungsgemäßen Phenylalkylaminen, wobei die Umsetzung der Aldehyde III unter reduzierenden Bedingungen stattfindet.

Die Umsetzung zwischen (II) und (IV) verläuft spontan, mit oder ohne Lösungsmittel bei einer Temperatur i. a. zwischen 50 und 200 °C, bevorzugt bei 130 bis 170 °C. Geeignete Lösungsmittel sind THF, Toluol, Xylol, DMF, Dimethylacetamid. Manchmal ist es vorteilhaft, ein neutralisierendes Mittel wie $Na_2CO_3$, $K_2CO_3$, $NaHCO_3$ zum Reaktionsgemisch zuzusetzen.

Herstellungsbeispiele für Vorprodukte

A. 2,4-Dimethyl-4-phenyl-butylchlorid

a) Zu einer Lösung 402 g 2-Phenylpropanal-1 und 12 g Ätznatron in 1 l Methanol werden innerhalb von 6 Stunden 192 g Propionaldehyd zugetropft. Man rührt noch eine Stunde, neutralisiert mit Eisessig (pH = 6), rührt über Nacht weiter, engt ein, nimmt mit $CH_2Cl_2/H_2O$ auf und extrahiert mit $CH_2Cl_2$. Man wäscht mit Wasser, trocknet über $Na_2SO_4$, engt wieder ein und destilliert. Man erhält 178 g 2,4-Dimethyl-4-phenylbut-2-en-1-al. Sdp. : 108 bis 120 °C/0,5 mbar.

b) Den erhaltenen Aldehyd (178 g 2,4-Dimethyl-4-phenyl-but-2-en-1-al) hydriert man in 600 ml Methanol mit 40 g Raney-Nickel bei 60 bis 70 °C und 100 bar Wasserstoffdruck. Nach Abfiltrieren des Katalysators wird eingeengt und destilliert. Man erhält 137 g 2,4-Dimethyl-4-phenyl-butanol-1. Die Umsetzungen werden zur Beschaffung eines größeren Vorrats wiederholt.

c) Zu 161 g Thionylchlorid werden 219 g 2,4-Dimethyl-4-phenyl-butanol-1 zugetropft. Man rührt über Nacht bei Raumtemperatur, anschließend 2 h bei 140°. Destillation des Rohprodukts ergibt 150 g 2,4-Dimethyl-4-phenyl-butylchlorid. Sdp. : 84 bis 86 °C/0,1 mm.

B. 2-Methyl-5-phenylpentylchlorid

Zu einer Suspension aus 0.45 Mol Phenylethylmagnesiumchlorid in 500 ml THF wird bei —15 °C 0,26 Mol 3-Brom-2-methylpropylchlorid in 50 ml THF zugetropft. Anschließend tropft man bei —15 °C 13 ml einer 0.5 molaren Lösung von $Li_2CuCl_4$ in THF zu.

Nach Erwärmen auf Raumtemperatur und Zugabe von 360 ml ges. $NH_4Cl$-Lösung wird mit Ether extrahiert, über $Na_2SO_4$ getrocknet und eingeengt. Destillation des Rückstandes ergibt 30 g 2-Methyl-5-phenylpentylchlorid. Sdp. : 83 bis 87 °C/0.1 mbar (wird wiederholt).

C. 5-p-tert.-Butylphenyl-2-methyl-pentylchlorid

Zu einer Mischung von 52 g 2-Methyl-5-phenyl-pentylchlorid und 4,9 g $FeCl_3$ werden unter Eiskühlung 27 g tert.-Butylchlorid zugetropft. Man rührt 7 h bei 50 °C und 12 h bei Raumtemperatur, nimmt in Methylenchlorid auf, wäscht mit Wasser, trocknet über $Na_2SO_4$ engt ein und destilliert. Man erhält 40 g 5-p-tert.-Butylphenyl-2-methyl-pentylchlorid. Sdp. : 114 bis 118 °C (0.2 mbar).

Nach den vorstehenden Beispielen lassen sich aus bekannten Vorprodukten die in der folgenden Tabelle zusammengestellten Phenylalkylchloride der Formel (II) herstellen.

(Siehe Tabelle 1 Seite 5 ff.)

Tabelle 1

Phenylalkylhalogenide

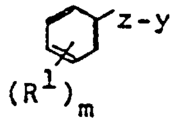

| Verb. Nr. | $(R^1)_m$ | - z - | y | Sdp °C/mbar |
|---|---|---|---|---|
| 1 | H | $(CH_2)_4$ | Br | 76° 0,4 |
| 2 | 4-tBu | $(CH_2)_4$ | Cl | |
| 3 | 4-Br | $(CH_2)_4$ | Cl | |
| 4 | H | $CHCH_3CH_2CHCH_3CH_2$ | Cl | 84 - 6° 0,1 |
| 5 | 4-Acetyl | $(CH_2)_4$ | Br | |
| 6 | 4-$OCH_3$ | $(CH_2)_5$ | Cl | |
| 7 | H | $(CH_2)_5$ | Br | 78 - 80° 0,2 |
| 8 | 4-t-Bu | $(CH_2)_5$ | Cl | 112 - 115° 0,4 |
| 9 | 4-Cl | $(CH_2)_5$ | Cl | 106 - 112° 0,2 |
| 10 | 4-$CH_3$ | $(CH_2)_5$ | Cl | 116 - 122° 0,8 |
| 11 | H | $(CH_2)_2CHCH_3(CH_2)_2$ | Cl | 80 - 82° 0,2 |
| 12 | H | $(CH_2)_3CHCH_3CH_2$ | Cl | 83 - 87° 0,2 |
| 13 | 4-t-Bu | $(CH_2)_3CHCH_3CH_2$ | Cl | 120 - 2° 0,2 |
| 14 | 2-$CH_3$ | $(CH_2)_5$ | Cl | 90 - 91° 0,2 |
| 15 | 4-Cyclohexyl | $(CH_2)_5$ | Br | |
| 16 | 4-Propionyl | $(CH_2)_5$ | Cl | |
| 17 | H | $(CH_2)_6$ | Cl | 86 - 90° 0,3 |
| 18 | H | $(CH_2)_4CHCH_3CH_2$ | Cl | 116 - 120° 0,2 |
| 19 | 4-Br | $(CH_2)_6$ | Cl | |
| 20 | 4-$OCH_3$ | $(CH_2)_6$ | Cl | |

Tabelle 1 (Fortsetzung)

| Verb. Nr. | $(R^1)_m$ | - z - | y | Sdp °C/mbar |
|---|---|---|---|---|
| 21 | 4-OtBu | $(CH_2)_6$ | Cl | |
| 22 | 4-Cyclohexyl | $(CH_2)_6$ | Cl | |
| 23 | 4-tBu | $(CH_2)_4CHCH_3CH_2$ | Cl | 130° 0,1 mm |
| 24 | 4-tBu | $(CH_2)_6$ | Cl | 120 - 2° 0,2 mm |
| 25 | 4-tBu | $CH_2CHCH_3(CH_2)_4$ | Cl | |
| 26 | 4-$CH_3$ | $CH_2CHCH_3(CH_2)_4$ | Cl | |
| 27 | 4-t-Amyl | $CH_2CHCH_3(CH_2)_4$ | Cl | |
| 28 | 2,4-$Cl_2$ | $CH_2CHCH_3(CH_2)_4$ | Cl | |
| 29 | 2,3,4-$Cl_3$ | $CH_2CHCH_3(CH_2)_4$ | Cl | |
| 30 | H | $(CH_2)_7$ | Cl | 116 - 122° 0,1 |
| 31 | 4-tBu | $(CH_2)_7$ | Cl | |
| 32 | H | $(CH_2)_{10}$ | Cl | |

Die Phenylalkylhalogenide der Formel (II) können unter entsprechender Abwandlung der folgenden Vorschrift zu den Phenylalkylaminen der Formel (I) umgesetzt werden.

Herstellungsbeispiel für eine erfindungsgemäße Verbindung

N-(2-Methyl-5-phenyl-pentyl)-2,6-cis-dimethylmorpholin

20 g 2-Methyl-5-phenylpentylchlorid und 35 g 2,6-cis-Dimethylmorpholin werden 10 h auf 150 °C erwärmt. Man läßt abkühlen, nimmt mit $CH_2Cl_2$ auf, wäscht mit Wasser, verdünnter NaOH und wieder mit Wasser. Nach Trocknen über $Na_2SO_4$ wird eingeengt und destilliert. Sdp. : 130 bis 2 °C/0,4 mbar. 18 g Ausbeute.

Entsprechend können die Phenylalkylhalogenide, beispielsweise die in Tabelle 1 angegebenen, zu erfindungsgemäßen Phenylalkylaminen der Formel I (Tabelle 2) umgesetzt werden ; soweit sie durch physikalische Angaben charakterisiert sind, wurden sie hergestellt und auf ihre biologische Wirkung untersucht. Die nicht charakterisierten Verbindungen lassen aufgrund struktureller Ähnlichkeit eine vergleichbare Wirkung erwarten.

(Siehe Tabelle 2 Seite 7 f.)

Tabelle 2

Phenylalkylamine

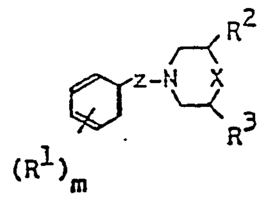

| Verb. Nr. | $(R^1)_m$ | - z - | X | $R^2$ | $R^3$ | Sdp °C/mbar |
|---|---|---|---|---|---|---|
| 101 | H | $(CH_2)_4$ | $CH_2$ | H | H | 116 - 120°/0,3 (bekannt aus EP-A-2,604, Beispiel 26) |
| 102 | 4-tBu | $(CH_2)_4$ | O | $CH_3$ | $CH_3$ | |
| 103 | 4-Br | $(CH_2)_4$ | O | $CH_3$ | $CH_3$ | |
| 104 | H | $(CH_2)_5$ | O | $CH_3$ | $CH_3$ | 134 - 6°/0,1 m |
| 105 | H | $(CH_2)_5$ | $CH_2$ | H | $CH_3$ | |
| 106 | 4-$OCH_3$ | $(CH_2)_5$ | $CH_2$ | H | H | |
| 107 | H | $(CH_2)_5$ | O | $C_2H_5$ | $C_2H_5$ | |
| 108 | H | $(CH_2)_3CHCH_3CH_2$ | O | $CH_3$ | $CH_3$ | 130 - 2°/0,4 |
| 109 | 4-Cl | $(CH_2)_5$ | - | H | H | |
| 110 | 4-tBu | $(CH_2)_5$ | O | $CH_3$ | $CH_3$ | 165 - 8°/0,3 |
| 111 | 4-tBu | $(CH_2)_3CHCH_3CH_2$ | O | $CH_3$ | $CH_3$ | 149 - 153°/0.2 |
| 112 | 4-tBu | $(CH_2)_3CHCH_3CH_2$ | S | $CH_3$ | $CH_3$ | |
| 113 | 4-tBu | $(CH_2)_3CHCH_3CH_2$ | $CH_2$ | $CH_3$ | $CH_3$ | |
| 114 | 4-tBu | $CH_2\overset{CH_3}{CH}CH_2\overset{CH_3}{CH}-CH_2$ | O | $CH_3$ | $CH_3$ | |
| 115 | 4-tBu | $CH_2\overset{CH_3}{CH}CH_2\overset{CH_3}{CH}-CH_2$ | $CH_2$ | $CH_3$ | $CH_3$ | 166 - 172°/0.1 |
| 116 | 4-$CH_3$ | $(CH_2)_5$ | O | $CH_3$ | $CH_3$ | 140 - 2°/0.3 |

Tabelle 2 : Fortsetzung

| Verb. Nr. | $(R^1)_m$ | - Z - | X | $R^2$ | $R^3$ | Sdp °C/mbar |
|---|---|---|---|---|---|---|
| 117 | 4-Cyclo-hexyl | $(CH_2)_5$ | O | $CH_3$ | $CH_3$ | |
| 118 | 4-Acetyl | $(CH_2)_4$ | O | $CH_3$ | $CH_3$ | |
| 119 | H | $(CH_2)_6$ | O | $CH_3$ | $CH_3$ | 128 - 130°/0.1 |
| 120 | H | $(CH_2)_6$ | $CH_2$ | H | $CH_3$ | |
| 121 | H | $(CH_2)_4CHCH_3CH_2$ | O | $CH_3$ | $CH_3$ | 135 - 8°/0.2 |
| 122 | 4-OtBu | $(CH_2)_6$ | O | $CH_3$ | $CH_3$ | |
| 123 | 4-Cyclo-hexyl | $(CH_2)_6$ | O | $CH_3$ | $CH_3$ | |
| 124 | 4-tBu | $(CH_2)_6$ | O | $CH_3$ | $CH_3$ | 162 - 5°/0.2 |
| 125 | 4-tBu | $(CH_2)_6$ | O | H | H | |
| 126 | 4-tBu | $(CH_2)_6$ | $CH_2$ | H | H | |
| 127 | 4-tBu | $(CH_2)_4CHCH_3CH_2$ | O | $CH_3$ | $CH_3$ | 159 - 160°/0.2 |
| 128 | 4-tBu | $(CH_2)_4CHCH_3CH_2$ | $CH_2$ | H | H | |
| 129 | 4-tBu | $CH_2CHCH_3(CH_2)_4$ | O | $CH_3$ | $CH_3$ | |
| 130 | 4-t-Amyl | $CH_2CHCH_3(CH_2)_4$ | O | $CH_3$ | $CH_3$ | |
| 131 | 2,4-$Cl_2$ | $CH_2CHCH_3(CH_2)_4$ | O | $CH_3$ | $CH_3$ | |
| 132 | 2,3,4-$Cl_3$ | $CH_2CHCH_3(CH_2)_4$ | O | $CH_3$ | $CH_3$ | |
| 133 | H | $(CH_2)_7$ | O | $CH_3$ | $CH_3$ | 148 - 152°/0.2 |
| 134 | 4-tBu | $(CH_2)_7$ | $CH_2$ | H | H | |

Tabelle 2 (Fortsetzung)

| Verb. Nr. | $(R^1)_m$ | - Z - | X | $R^2$ | $R^3$ | Sdp °C/mbar |
|---|---|---|---|---|---|---|
| 135 | 4-tBu | $(CH_2)_7$ | O | $CH_3$ | $CH_3$ | |
| 136 | H | $(CH_2)_{10}$ | O | $CH_3$ | $CH_3$ | |
| 137 | 4-tBu | $(CH_2)_2CHCH_3(CH_2)_2$ | O | $CH_3$ | $CH_3$ | 145-150/0.1 |
| 138 | H | $(CH_2)_3CHC_2H_5CH_2$ | O | $CH_3$ | $CH_3$ | 160-162/0.7 |
| 139 | H | $CHCH_3CH_2CHC_2H_5CH_2$ | O | $CH_3$ | $CH_3$ | 128-133/0.5 |
| 140 | H | $CHCH_3CH_2CHCH_3CH_2$ | O | $CH_3$ | $CH_3$ | 134-138/0.7 |
| 141 | H | $(CH_2)_2(CHCH_3)_2CH_2$ | O | $CH_3$ | $CH_3$ | 140-142/0.7 |

Auf folgende Weise kann z. B. die wachstumsregulierende Wirkung der neuen Phenylalkylamine bestimmt werden.

1. Reiskeimlingstest

Die Testdurchführung ist beschrieben in : W. Rademacher und J. Jung, Z. Acker- und Pflanzenbau 150, 363-371 (1981). Dieser Test dient zur Charakterisierung wachstumsregulatorischer Aktivität. Die Verbindungen 110, 111 und 124 zeigten eine den Standards Chlormequatchlorid und Fenpropimorph deutlich überlegene einkürzende Wirkung.

Wirkstoffkonzentrationen (Mol/l), die bei Reiskeimlingen (Sorte Girona) zu einer 50 %igen Einkürzung der zweiten Blattscheide führen

| Wirkstoff | Konzentration |
|---|---|
| Chlormequatchlorid | $1,5 \times 10^{-2}$ |
| Fenpropimorph | $1,0 \times 10^{-4}$ |
| Verbindung 110 | $1,4 \times 10^{-5}$ |
| Verbindung 111 | $3,5 \times 10^{-5}$ |
| Verbindung 116 | $1,9 \times 10^{-5}$ |
| Verbindung 124 | $2,7 \times 10^{-5}$ |

2. Sonnenblumen - Nachauflaufverfahren

Sonnenblumen der Sorte Spanners Allzweck wurden auf ausreichend mit Nährstoffen versorgtem Kultursubstrat in Kunststoffgefäßen von ungefähr 12,5 cm Durchmesser angezogen. Im Nachauflaufverfahren wurden die zu prüfenden neuen Verbindungen in wäßriger Aufbereitung auf die Pflanzen versprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt.

Bei Behandlung mit z. B. 6 mg Wirkstoff/Gefäß zeigten die Amine 110, 111, 121, 127 und 137 eine den Standards Cycocel und Fenpropimorph überlegene einkürzende Wirkung :

| Wirkstoff | Sproßhöte (% von Kontrolle) |
|---|---|
| Chlormequatchlorid | 87,5 |
| Fenpropimorph | 81,0 |
| Verbindung 110 | 74,5 |
| Verbindung 111 | 71,3 |
| Verbindung 121 | 71,3 |
| Verbindung 127 | 76,1 |
| Verbindung 137 | 70,7 |
| Kontrolle | 100 |

3. Saatgutbehandlung Weizen, Gerste, Mais

Saatgutbehandlung bei Weizen (Sorte Kolibri), Gerste (Sorte tramis), Mais (Sorte Inrakorn) mit 1 g

Wirkstoff je kg Saatgut führte zu starker Wuchshöhenverminderung. Dabei erwies sich insbesondere Ver. Nr. 111 den Standards Chlormequatchlorid und Fenpropimorph überlegen.

(Angaben in % der jeweiligen Kontrolle)

| Wirkstoff | Weizen | Gerste | Mais |
|---|---|---|---|
| Chlormequatchlorid | 87 | 96 | 100 |
| Fenpropinmorph | 92 | 91 | 101 |
| Verbindung 111 | 24 | 57 | 63 |

**Patentansprüche**

1. Phenylalkylamine der Formel

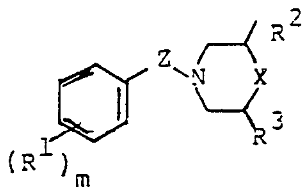

(I)

in der

$R^1$ bis zu drei gleiche oder verschiedene Substituenten, ausgewählt aus $CH_3$, $C_2H_5$, n-Propyl, iso-propyl, n-Butyl, iso-Butyl, tert.-Butyl, tert.-Amyl, 1,1-Dimethyl-butyl, 1,1-Dimethyl-pentyl, Cyclopentyl, Cyclohexyl, Cycloheptyl, Norbornyl, Methoxy, Ethoxy, Propoxy, tert.-Butoxy, Acetyl, Propionyl, Benzoyl, Pivaloyl oder Halogen,

$R^2$ und $R^3$ Wasserstoff oder $C_1$- bis $C_3$-Alkyl,

z geradkettiges $C_5$- bis $C_{10}$-Alkylen, gegebenenfalls mit bis zu drei $C_1$- bis $C_4$-Alkyl substituiert, oder $CH_2—CH(CH_3)CH_2CH_2$,

X Sauerstoff, Schwefel, $(CH_2)_p$,

m die Zahlen 0 bis 3 und

p die Zahlen 0 bis 2 bedeuten.

2. Phenylalkylamine der Formel I nach Anspruch 1, in der $R^2$ und $R^3$ jeweils Methyl und X Sauerstoff sind und $R^1$, z und m die oben genannten Bedeutungen haben.

3. Verfahren zur Herstellung der Verbindungen gemäß Anspruch 1 oder 2, dadurch gekennzeichnet, daß man ein entsprechendes Phenylalkylhalogenid der Formel

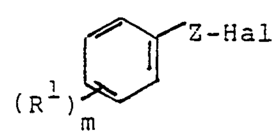

(II)

oder ein entsprechendes ω-Phenylalkanal der Formel

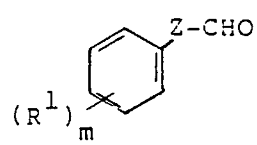

(III)

umsetzt mit einem entsprechenden Amin der Formel

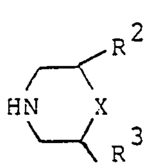

(IV)

wobei die Umsetzung der Aldehyde III unter reduzierenden Bedingungen staffindet.

4. Mittel zur Regulierung des Pflanzenwachstums, enthaltend ein Phenylalkylamin gemäß Anspruch 1 oder 2 und wenigstens einen Hilfsstoff.

5. Verfahren zur Regulierung des Pflanzenwachstums, dadurch gekennzeichnet, daß man eine Verbindung der Formel I gemäß Anspruch 1, in der $R^1$, $R^2$, X, m und p die in Anspruch 1 genannten Bedeutungen haben und z geradkettiges $C_4$- bis $C_{10}$-Alkylen, gegebenenfalls mit bis zu drei $C_1$-$C_4$-Alkyl substituiert, auf die Pflanzen oder deren Vermehrungsgut einwirken läßt.

## Claims

1. A phenylalkylamine of the formula

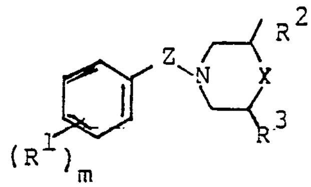

(I)

where the radicals

$R^1$ are not more than three identical or different substituents and are each selected from the group consisting of $CH_3$, $C_2H_5$, n-propyl, isopropyl, n-butyl, isobutyl, tert.-butyl, tert.-amyl, 1,1-dimethylbutyl, 1,1-dimethylpentyl, cyclopentyl, cyclohexyl, cycloheptyl, norbornyl, methoxy, ethoxy, propoxy, tert.-butoxy, acetyl, propionyl, benzoyl, pivaloyl and halogen,

$R^2$ and $R^3$ are each hydrogen or $C_1$-$C_3$-alkyl,

z is straight-chain $C_5$-$C_{10}$-alkylene which is unsubstituted or substituted by not more than three $C_1$-$C_4$-alkyl radicals or is $CH_2$—$CH(CH_3)CH_2CH_2$,

X is oxygen, sulfur or $(CH_2)p$,

m is from 0 to 3 and

p is from 0 to 2.

2. A phenylalkylamine of the formula I as claimed in claim 1, wherein $R^2$ and $R^3$ are each methyl, X is oxygen and $R^1$, z and m each have the above meanings.

3. A process for the preparation of a compound as claimed in claim 1 or 2, wherein an appropriate phenylalkyl halide of the formula

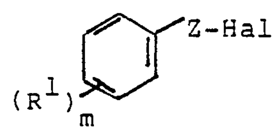

(II)

or an appropriate ω-phenylalkanal of the formula

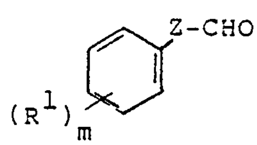

(III)

is reacted with an appropriate amine of the formula

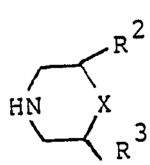

(IV)

the reaction with the aldehyde III taking place under reducing conditions.

4. A plant growth regulator which contains a phenylalkylamine as claimed in claim 1 or 2 and one or more assistants.

5. A method of regulating plant growth, wherein a compound of the formula 1 as claimed in claim 1 where $R^1$, $R^2$, X, m and p each have the meanings stated in claim 1 and z is straight-chain $C_4$-$C_{10}$-alkylene which is unsubstituted or substituted by not more than 3 $C_1$-$C_4$-alkyl radicals, is allowed to act on the plants or on their propagation stock.

**Revendications**

1. Phénylakylamines de formule

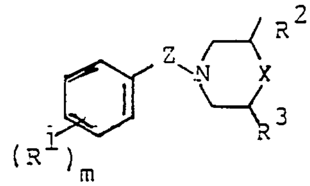

(I)

dans laquelle

$R^1$ représente jusqu'à trois substituants identiques ou différents choisis parmi $CH_3$, $C_2H_5$, n-propyle, iso-propyle, n-butyle, iso-butyle, tert.-butyle, tert.-amyle, 1,1-diméthyl-butyle, 1,1-diméthyl-pentyle, cyclopentyle, cyclohexyle, cycloheptyle, norbornyle, méthoxy, ethoxy, propoxy, tert.-butoxy, acétyle, propionyle, benzoyle, pivaloyle ou halogène

$R^2$ et $R^3$ représentent hydrogène ou alkyle en $C_1$ à $C_3$

Z représente alkylène en $C_5$ à $C_{10}$ à chaîne droite, éventuellement substitué avec jusqu'à trois alkyle en $C_1$ à $C_4$ ou $CH_2$—$CH(CH_3)CH_2CH_2$

X représente oxygène, soufre, $(CH_2)_p$

m représente les nombres 0 à 3 et

p les nombres 0 à 2

2. Phénylalkylamines de formule I selon la revendication 1, dans laquelle $R^2$ et $R^3$ sont chacune méthyle et X oxygène, et $R^1$ Z et m ont les significations sus-indiquées.

3. Procédé de préparation de composés selon la revendication 1 ou 2, caractérisé par le fait que l'on fait réagir un phénylalkylhalogénure correspondant de formule

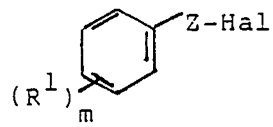

(II)

ou un ω-phénylalcanal correspondant de formule

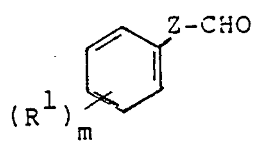

(III)

avec une amine correspondante de formule

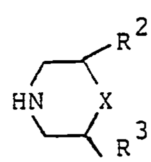

(IV)

la réaction de l'aldéhyde III s'opérant sous des conditions réductrices.

4. Moyen de régulation de la croissance des plantes, contenant une phénylalkylamine selon la revendication 1 ou 2 et au moins une urée.

5. Procédé de régulation de la croissance des plantes, caractérisé par le fait que l'on fait agir sur les plantes ou leurs produits de prolifération, un composé de formule I selon la revendication 1 dans laquelle $R^1$, $R^2$, X, m et p ont les significations indiquées dans la revendication 1 et Z alkylène en $C_4$-$C_{10}$ à chaîne droite, éventuellement substitué avec jusqu'à trois alkyle en $C_1$-$C_4$.